# EUROPÄISCHE PATENTANMELDUNG

(11) **EP 4 275 694 A1**
(43) Veröffentlichungstag der Anmeldung: **15.11.2023**
(21) Anmeldenummer: 22173218.3
(22) Anmeldetag: 13.05.2022
(51) Int. Cl.: A61K 36/185, A61K 36/11, A61K 36/23, A61K 36/28, A61K 36/38, A61K 36/48, A61K 36/73, A61K 36/86, A61K 36/9066, A61P 17/00, A61K 8/00, A61K 9/00, A61K 9/06

(54) **VERWENDUNG VON SOLUBILISATEN IN ERZEUGNISSEN ZUR TOPISCHEN ANWENDUNG**

(71) Anmelder: Solmic Biotech GmbH, 40225 Düsseldorf (DE)
(72) Erfinder: FURCH, Marcus, 65719 Hofheim am Taunus (DE); LOSA, Anastasia, 47877 Willich (DE)
(74) Vertreter: Schüssler, Andrea

(57) **Zusammenfassung**

Die Erfindung betrifft eine Zusammensetzung zur topischen Anwendung für medizinische oder kosmetische Zwecke, die mizellierte Extrakte oder mizellisierte lipophile Wirkstoffe enthält, insbesondere eine Zusammensetzung aufweisend einen oder mehrere Solubilisate von lipophilen Wirkstoffen aus Pflanzenauszügen, Pflanzenextrakten, Pflanzenteilen, Phytotherapeutika, Harzen, Vitaminen, Enzymen, Proteinen, Algen, funktionellen Aktivstoffen und/oder Coenzymen sowie Mischungen und Kombinationen davon.

## Beschreibung

Die Erfindung betrifft Erzeugnisse zur topischen Anwendung aufweisend mizellierte Extrakte oder mizellisierte lipophile Wirkstoffe.

Die Einarbeitung von lipophilen und in Wasser kaum oder nicht löslichen Wirkstoffen in Endprodukte stellt insofern ein Problem dar, als solche Wirkstoffe zwecks homogener Verteilung erst nach einem entsprechend zeit- und kostenaufwendigen Verfahren unter Verwendung von Öl/Öl-Gemischen bzw. Öl-Wasser-Emulsionen in die Endprodukte eingearbeitet werden können. Um eine fettlösliche Substanz in das Endprodukt einzuarbeiten, muss diese durch eine unerwünscht große Menge an Öl volumenmäßig vergrößert werden, damit eine optimale homogene Verteilung im Endprodukt gewährleistet ist.

In der EP 1 768 497 B1 wird daher die Herstellung von Solubilisaten von ätherischen Ölen beschrieben, um in Wasser nicht oder schwerlösliche Substanzen homogen in Endprodukte einbringen zu können.

In der EP 3 820 528 A1 werden Solubilisate mit Curcumin und zumindest einem Cannabinoid als weiterem Wirkstoff beschrieben, um daraus Kapseln herzustellen, die als Nahrungsergänzungsmittel bzw. Arzneimittel wirken.

WO 2021/037656 A1 beschreibt Folsäure-Solubilisate, die in Nahrungsergänzungsmitteln und in der Abwasserbehandlung in Klärablagen Verwendung finden.

In der WO 2021/175763 A1 werden topische Zusammensetzungen beschrieben, die Cannabidiol, das in Niosomen eingeschlossen ist, beinhalten. Die Niosomen bestehen aus einer hydrophoben Doppelschicht und einem hydrophilen Kern, d.h. fettlösliche Substanzen werden zwischen den Doppelschichten eingeschlossen, während wasserlösliche Substanzen im Inneren des Kerns eingeschlossen werden können.

WO 2021/030789 A1 betrifft nanoverkapseltes Cannabidiol, das allein oder zusammen mit Naltrexon als Therapeutikum bei einer Anzahl von Krankheiten, wie Schmerzzuständen, Parkinson-Krankheit, Alzheimer-Krankheit, Multiple Sklerosis, Morbus Crohn und Entzündungen, eingesetzt wird.

Der vorliegenden Erfindung liegt die Aufgabe zu Grunde, Erzeugnisse zur topischen Anwendung zur Verfügung zu stellen, in denen pflegende oder heilende Wirkstoffe für die Haut so aufbereitet sind, dass sie von der Haut gut aufgenommen werden und dort zu einer Verbesserung des Hautbildes führen. Desweiteren sollen sich die Erzeugnisse durch eine gute Stabilität und Haltbarkeit auszeichnen

Diese Aufgabe wird bei einem Erzeugnis zur topischen Anwendung dadurch gelöst, dass es Solubilisate von lipophilen Wirkstoffen enthält.

Die Erfindung betrifft somit ein Erzeugnis bzw. eine Zusammensetzung zur topischen Anwendung, welche(s) Solubilisate von lipophilen Wirkstoffen wie in Anspruch 1 definiert, enthält. Somit werden die Solubilisate der lipophilen Wirkstoffe wie in Anspruch 1 definiert, dazu verwendet, Erzeugnisse bzw. Zusammensetzungen zur topischen Anwendung herzustellen.

Im Rahmen der Anmeldung werden die Begriffe "Erzeugnis(se)", "Produkt(e)" und "Zusammensetzung(en)" synonym verwendet.

Die Erzeugnisse enthalten solubilisierte Wirkstoffe, die verglichen mit den reinen Isolaten der Wirkstoffe, besser in das Produkt verarbeitet werden können und dort zu einer verbesserten Stabilität beitragen.

Die Solubilisate sind wasserlösliche Mizellen, die lipophile Stoffe verpacken. Solubilisate werden mit dem Ziel hergestellt, lipophile oder nicht bzw. schlecht wasserlösliche Substanzen so einzuschließen, dass sie wasserlöslich werden. Eine Kombination von Emulgator(en), fettlöslichen Wirkstoffen und Hilfsstoffen bildet stabile, transparente und wasserlösliche Solubilisate. Als Emulgatoren werden bevorzugt Polysorbate, Poloxamer (Lutrol), Gummi Arabicum, Sucrosestearat, DermofeelG 10 LW(70) MB, pastose Dermofeel^{®} G 10 L, PEG-40 hydriertes Rizinusöl (hydrogenated castor oil), Lecithine, Phospholipon, oder D-alpha-Tocopheryl Polyethylenglycol 1000 Succinat (TPGS; andere Bezeichnung: Vitamin E TPGS, Tocofersolan (INN)) eingesetzt. Beispielsweise sind Poloxamere geeignete Emulgatoren. Poloxamere sind synthetische Block-Copolymere aus Ethylenoxid und Propylenoxid. Es existieren verschiedene flüssige und halbfeste Typen, die in Wasser löslich bis sehr leicht löslich sind. Poloxamere sind amphiphil. Beispielsweise ist TPGS ein anderer amphiphiler Emulgator, der einen hydrophilen und einen lipophilen Molekülteil aufweist. Ein Polyethylenglykolanteil stellt den polaren Kopf und das Tocopherolsuccinat den lipophilen Schwanz dar. Diese amphiphile Eigenschaft führen zur Selbstassoziierung (Micellenbildung) von TPGS in Wasser. Somit werden fettlösliche Bestandteile in das Innere der Micelle (liphophiler Teil) eingeschlossen und durch die wasserlösliche Oberfläche der Micelle in Wasser gelöst. Mögliche Hilfsstoffe zur besseren Löslichkeit der schwer wasserlöslichen Stoffe sind z.B. Ethanol (96%), Polysorbat, Glycerin (wasserfrei). Für weitere Details zur Herstellung der Solubilisate sei auf den Inhalt die EP-Anmeldungen EP 3 675 825 (EP 18 738 297) und EP 3 675 824 (EP 18 737 274) der Anmelderin verwiesen.

Die stabil in Mizellen eingeschlossenen Wirkstoffe sind vor thermischen, Licht-Einflüssen und/oder chemischer Instabilität (z.B. Oxidation) geschützt. Die Mizellen zeichnen sich dadurch aus, dass ein hydrophober Kern, in dem die fettlöslichen Substanzen eingeschlossen sind, von einer hydrophilen Emulgatoren-Schicht umgeben sind.

In der Herstellung der Erzeugnisse wird das Solubilisat als einzelner Rohstoff verwendet. Das hat den Vorteil, dass es direkt in die Wasserphase eingebaut werden kann bzw. die Wasserphase komplett ersetzen kann. Somit können nicht nur Ö/W (Öl in Wasser) sondern auch W/Ö (Wasser in Öl) als auch reine wässrige Erzeugnisse ohne eine Zugabe von Fett/Öl oder Emulgatoren erzeugt werden.

Die wasserlöslichen Mizellen verteilen sich gleichmäßig in der wässrigen Lösung. Dadurch wird die Homogenität des Wirkstoffes im Produkt erreicht. Zudem werden in der Mizelle verpackte hydrophobe Wirkstoffe (Extrakte, lipophile Vitamine, etc.) von äußeren Einflüssen in der Mizelle geschützt. Damit wird eine verbesserte Stabilität des Wirkstoffes im Produkt gewährleistet.

Erzeugnisse zur topischen Anwendung sind vorrangig Kosmetikprodukte, aber auch medizinische oder pharmazeutische Zusammensetzungen zur Applikation auf die Haut oder den Schleimhäuten. Beispiele sind Cremes, Lotionen, Milche, Seren, Gele, Tropfen, Emulsionen, Balsame, Fluide, Shampoos, Salben, Masken, Schäume, Sprays oder Gesichtswasser. Diese Erzeugnisse können sowohl auf wässriger Basis als auch auf Fett/Öl-Basis sein. Die Erzeugnisse können der Pflege, Reinigung und/oder therapeutischen Behandlung der Haut bzw. den Schleimhäuten von Kopf bis Fuß dienen. Besonders bevorzugt sind Gesichtscremes und -seren.

Als Wirkstoffe kommen lipophile Pflanzenauszüge, Pflanzenextrakte, Phytotherapeutika, Algen, Harze, Vitamine, Enzyme, Proteine, funktionelle Aktivstoffe und/oder Coenzyme sowie Mischungen und Kombinationen davon in Frage. Allen genannten Wirkstoffen ist ihre positive Wirkung auf die Haut gemein, die sowohl die kosmetische Anwendung zur Pflege und Reinigung jeglichen Hauttyps, insbesondere zur Erreichung eines Anti-Aging Effekts, als auch therapeutische Anwendungen bei Hauterkrankungen, wie Akne, Erysipel, Urtikaria, Herpes, Neurodermitis, Verbrennungen oder Psoriasis, umfasst.

Beispiele für mögliche Pfanzenauszüge oder Pflanzenextrakte bzw. Phytotherapeutika sind Nachtkerze (Oenothera biennis), Ballonrebe (Cardiospermum halicacabum), Wildes Stiefmütterchen (Viola tricolor), Zinnkraut (Equisetum arvense), Echte Kamille (Matricaria recutita), Zauberstrauch (Hamamelis virginiana), Mahonie (Mahonia aquifolium), Birke (Betula pendula), Ylang-Ylang, Sibirischer Ginseng (Eleutherocokk), Sheabutter (Karitebutter), Cannabispflanze (Cannabinoide), Kurkuma (Curcuma langa), Echter Lavendel (Lavandula angustifolia), Johanniskraut (Hypericum perforatum), Irisches Moos (Chondrus Crispus), Hopfen (Humulus Lupulus) Grüntee (Camellia sinensis L.), Centella ( Centella asiatica), Malve (Malva sylvestris), Mädesüß (Filipendula ulmaria), Ringelblume (Calendula officinalis L.) oder Rotklee (Trifolium pratense).

Beispiele für mögliche Algen sind Braunalge, Rotalge, Grünalge oder Blaualge.

Beispiele für mögliche Vitamine sind Vitamin E, Vitamin A, Pro-Vitamin A (Beta-Karotin), Vitamin C, Vitamin B 3 (Niacin), Panthenol, Provitamin B5 und Vitamin B7 (Biotin).

Beispiele für Enzyme, Proteine und Coenzyme sind Lipasen, Cellulasen, Amylasen, Proteasen, Kollagen, Acetyl-Hexapeptid-8, Pentapeptid-18 oder Ubichinon-10 (Q10), Beispiele für mögliche Harze sind Weihrauch, Propolis oder Myrrhe.

Beispiele für weitere funktionelle Aktivstoffe sind Fruchtsäuren (z.B. Glykolsäure, Milchsäure, Apfelsäure, Citronensäure, Weinsäure), Resveratrol, Pterostilbene, Astaxanthin oder Substanzen, die mit den mit den Funktionswegen von Hypoxieinduzierter Faktor (HIF), mTor, p53, GDNF, GLUT1 interagieren können.

Besonders bevorzugte Solubilisate sind:
mizelliertes CBD (Cannabidiol) -> Anwendung: anti-Falten, antientzündlich]
mizellisiertes Q10 oder Astaxanthin -> Anwendung: anti-Falten, Energy Booster, Antioxidans
mizellisierter Weihrauch -> Anwendung: antibakteriell, antientzündlich
mizellisierter Propolis -> Anwendung: antibakteriell, antientzündlich
mizellisierter Sibirischer Ginseng, Resveratrol, Pterostilbene oder HIF Inhibitoren -> Anwendung: anti-Aging, anti-Falten, antibakteriell, antientzündlich

Zu dem Solubilisat bzw. dem Produkt zur topischen Anwendung können ein oder mehrere weitere (wasserlösliche) Stoffe hinzugefügt sein, die eine positive Wirkung auf die Haut haben, z.B. Hyaluronsäure, Aloe Vera, Centella-Extrakt, Grüntee-Extrakt, Milchsäure, Harnstoff (Urea) oder Aminosäuren.

Des Weiteren können noch kosmetische bzw. pharmazeutische Hilfsstoffe, wie Stabilisatoren, Emulgatoren, Konservierungsmittel, Verdickungsmittel und/oder Bindemittel vorhanden sein.

Damit Öl und Wasser sich nicht abstoßen, benötigten alle Emulsionen für ihre Stabilität einen Emulgator bzw. Lösungsvermittler. Der Emulgator ist an der Grenzfläche zwischen beiden Flüssigkeiten angeordnet und hat sowohl einen polaren als auch einen unpolaren Molekülteil. Die polaren Enden der Emulgatormoleküle heften sich an das Wasser, die unpolaren Enden an die Ölmoleküle. Durch dieses "Andocken" setzen Emulgatoren die Grenzflächenspannung zwischen der Öl- und der Wasserphase herab und stabilisieren so die Emulsion. Geeignete Emulgatoren sind z.B. Laureth 4, Ceteareth -15, PEG-8, Sucrosestearate (E 473), Methylglucosesequistearate oder Glycerylstearate.

Oft werden den Emulgatoren noch Stabilisatoren zugesetzt. Diese umhüllen die emulgierten Tröpfchen mit einem Schutzfilm und sorgen für starken Zusammenhalt. Stabilisatoren sind beispielsweise antioxidative Vitamine, Stärke, Dextrine, Pektine oder Eiweißstoffe. Erfindungsgemäß bevorzugte Stabilisatoren (z.B. in Gesichtscremes oder -lotionen) sind Vitamin C oder Vitamin E, vorzugsweise in einer Menge von 0,2 - 1,8 Gew-%, ganz bevorzugt 0,5 - 1,0 Gew-%.

Verdickungsmittel in Kosmetikprodukten sorgen dafür, dass die Produkte immer eine optimale Konsistenz haben, und erhöhen die Viskosität. So machen sie beispielsweise Lotionen zähflüssiger und cremiger, was das Auftragen erleichtert und für ein sanftes Hautgefühl sorgt. Diese können synthetische Verdicker, wie flüssige Mikro-Plastik-Partikel, sein oder natürliche Verdicker wie Cellulose (E 460), Xanthangum (E 415), Algin (E 400 - E 405), Gellangum (E 418), Pektin (E 440) oder Carrageenan (E 407) sein. Die natürlichen Verdickungsmittel sind, obwohl sie teurer sind, klar bevorzugt, da Mikroplastik für die Umwelt ein klares Problem darstellt.

Konservierungsmittel schützen kosmetische Produkte vor dem mikrobiellen Verderb und leisten damit einen wichtigen Beitrag zur Gesundheit der Verbraucher. Wasserhaltige Produkte sind besonders gefährdet, da sich Mikroorganismen leicht ansiedeln und vermehren können. Oft verwendete Konservierungsmittel sind Parabene, Alkohole (z.B. Ethylalkohol) oder halogenorganische Stoffe (z.B. Benzoisothiazolinone, Methylisothiazolinone, Dichlorobenzylalkohol, Chlorhexidin).

Bindemittel als Inhaltsstoffe in Kosmetika sorgen für den gewünschten Zusammenhalt (Kohäsion) der Produkte. Sie verhindern, dass sich Feststoffe und flüssige Bestandteile entmischen. Üblicherweise liegen die Einsatzmengen unter 2% der Gesamtmasse. Häufig verwendet werden hydrophile Kolloide (z.B. Alginate) sowie Carboxymethylcellulose, Hydroxyethylcellulose oder Xanthan.

Erfindungsgemäß konnte gezeigt werden, dass bei Verwendung der Wirkstoff-Solubilisate, verglichen mit den Isolaten der Wirkstoffe, eine höhere Stabilität des Produkts zur topischen Anwendung erreicht werden kann. Dies gilt insbesondere für Gesichtscremes und Gesichtsseren, besonders solchen mit CBD als Wirkstoff. Die durch die Wirkstoff-Solubilisate erreichte Stabilität kann durch die Zugabe von Stabilisatoren noch gesteigert werden. Will man allerdings Stabilisatoren im Kosmetikprodukt vermeiden, sind die Solubilisate gegenüber den Isolaten klar überlegen, da die Solubilisate bereits zu einer Stabilität des Produkts beitragen, ohne weitere Stabilisatoren zu benötigen. Bei Zugabe von Vitamin E in höherer Konzentration (z.B. 1%) ist eine deutliche Stabilität des Solubilisats zu beobachten gegenüber der Stabilität des Isolates. Dieser Effekt ist wenigstens bei Cremes mit höherem Fettanteil auch weiterhin zu beobachten mit 1% Vitamin E plus 1% Vitamin C.

Aufgrund der wasserlöslichen Eigenschaft der Solubilisate, prädestinieren sich diese in der Anwendung in kosmetischen Mitteln, wo keine Fette oder Öle erwünscht sind, wie z.B. Shampoos oder Masken

Durch den Mizellenschutz wird der Wirkstoff in inneren Kern vor Lichteinstrahlung geschützt, dadurch profitiert die Produktstabilität im täglichen Gebrauch.

Die Erfindung wird anhand der Figuren näher erläutert:
- Fig. 1:: **CBD in Acetonitril/Wasser bei Tageslicht und RT**
- Fig. 2:: **CBD in Acetonitril/Wasser im Dunkel und 4°C**
- Fig. 3:: **CBD Poloxamer bei Tageslicht und RT**
- Fig. 4:: **CBD Poloxamer im Dunkel und 4 °C**

Die Erfindung wird durch das nachfolgende Beispiele erläutert.

### Beispiel 1

### Mikrobielle Stabilität des Solubilisats im kosmetischen Produkt CBD- Gel

### Zusammensetzung des Gels mit 1 % CBD-Solubilisat

### Herstellung:

Die entsprechende Menge an Solubilisat wird der Wasserphase zugesetzt. Die vorgelösten Gelbinder werden der Wasserphase zugegeben und bis zu eindicken des Gels gerührt. Weitere Wirkstoffe und Konservieren werden zugefügt.

Probe Nr.: COS011-AL-009

Bestandteile (Ingredienzen):
Rosenhydrolat, Propylenglykol, Wasser, Betain, Natrosol 250 HX, CBD-Solubilisat, Milchsäure, Kaliumsorbat, Xanthan

Das Gel, welches zu 80% aus wässriger Phase besteht, und somit das Wachstum von Keimen begünstigt, wurde dem Keimbelastungstest unterzogen

Methode:
Zur Bestimmung des Ausgangskeimgehaltes der Probe werden 1 g Probe mit Eugon LT SUP-Puffer in der Regel 1:10 verdünnt. Zur Prüfung der ausreichenden Konservierung wird die Probe mit 1% der jeweiligen Keimsuspension inokuliert. Die beimpften Proben werden bis zum jeweiligen Re-Isolierungszeitpunkt bei 22,5 ± 2,5 °C gelagert. Nach T7, T14 und T28 wird die Anzahl des jeweiligen Mikroorganismus im Oberflächenausstreichverfahren bestimmt. Bei Durchführung einer Wiederfindung werden je 1 g Probe mit der entsprechenden Keimsuspension im Verhältnis 1:10 einzeln beimpft. Im Kontrollansatz erfolgt die Beimpfung ohne Probe

Ergebnis:
1. Untersuchungsergebnisse / Analysis results:

**Tabelle / Table 1: Ergebnisse des Konservierungsbelastungstests / Results of the preservative efficacy test**

| **Probe/Sample** | **KBE/g Probe CFU/g sample** | | | | |
|---|---|---|---|---|---|
| **21-049260-01** | | | | | |
| Inkubationszeit / Incubation time | Staphylococcus aureus | Pseudomonas aeruginosa | Escherichia coli | Candida albicans | Aspergillus brasiliensis |
| Verdünnung/ Dilution | 1:10 | 1:10 | 1:10 | 1:10 | 1:10 |
| Inokulum/ Inocula | 2,1 ×10 ⁵ | 1,1 ×10 ⁵ | 1,5 ×10 ⁵ | 2,0 ×10 ⁴ | 1,1 ×10 ⁴ |
| T7 | <10 | <10 | <10 | 10 | - |
| T14 | <10 | <10 | <10 | <10 | <10 |
| T28 | <10 | <10 | <10 | <10 | <10 |

| | | | | | |
|---|---|---|---|---|---|
| T2, T7, T14, T28, T35, T42: Probenahmezeitpunkte / Sampling times | | | | | |

**Tabelle / Table 2: Keimreduktion / Reduction of germs**

| **Probe/Sample** | **Keimreduktion/ Reduction of germs (log(10))** | | | | |
|---|---|---|---|---|---|
| **21-049260-01** | | | | | |
| Inkubationszeit / Incubation time | Staphylococcus aureus | Pseudomonas aeruginosa | Escherichia coli | Candida albicans | Aspergillus brasiliensis |
| Inokulum/ Inocula | 5,32 | 5,04 | 5,18 | 4,30 | 4,04 |
| T7 | ≥ 3 | ≥ 3 | ≥ 3 | ≥ 1 | --- |
| T14 | ≥ 3 | ≥ 3 | ≥ 3 | ≥ 1 | ≥ 1 |
| T28 | ≥ 3 | ≥ 3 | ≥ 3 | ≥ 1 | ≥ 1 |

| | | | | | |
|---|---|---|---|---|---|
| T2, T7, T14, T28, T35, T42: Probenahmezeitpunkte / Sampling times | | | | | |

Das Produkt erfüllt in der Prüfung des antimikrobiellen Schutzes (Prüfung auf ausreichende Konservierung) Kriterium A und B gemäß Anhang B der DIN EN ISO 11930.

**Folgende Keime wurden benutzt:**

| **Prüforganismen / Germs ^{∗}** | **Stamm / Strain** | **Nährmedien / Nutrient media Bebrütungstemperatur / Incubation temperature Bebrütungsdauer / Incubation time** |
|---|---|---|
| Staphylococcus aureus | ATCC 6538 | Trypton Soja-Agar / Tryptone Soy Agar 32,5°C ± 2,5 °C 48 -72 h |
| Pseudomonas aeruginosa | ATCC 9027 | |
| Escherichia coli | ATCC 8739 | |
| Candida albicans | ATCC 10231 | Sabouraud-Dextrose-Agar / Sabouraud Dextrose Agar 32,5°C ± 2,5 °C 48 -72 h |
| Aspergillus brasiliensis | ATCC 16404 | Kartoffel-Dextrose-Agar / Potato Dextrose Agar 22,5°C ± 2,5°C 3 - 5 d |

| | | |
|---|---|---|
| ^{∗} Aufbewahrung in Stammsammlung / Storage in culture collection | | |

### Beispiel 2:

### Stabilitätsstudie mikrobiologische Untersuchung an Propolis-Solubilisat

Für die mikrobiologische Untersuchung wurde Propolis-Solubilisat, das aus dem ethanolischen Propolis-Extrakt hergestellt wurde, in 50 ml brauen Glasflaschen bei Raumtemperatur und 40 °C Klimaschrank gelagert. Es wurden zum Zeitpunkt Tag 0, 90 und 180 Proben gezogen und auf mikrobiologische Verkeimung untersucht.

| Mikrobiologie an Beispiel Propolis-Solubilisat: Sol070-IK-Mibi | | | | |
|---|---|---|---|---|
| Mikrobiologische Untersuchung. Probenzug t0, t90, t180 bei Raumtemperatur und 40 °C | | | | |
| | NG | T0 RT/40°C | T90 RT/40°C | T180 RT/40°C |
| Aerobe mesophile Gesamtkeimzahl | 1 | n.n | n.n | n.n |
| Hefen | 1 | n.n | n.n | n.n |
| Schimmelpilze | 1 | n.n | n.n | n.n |
| Escherichia coli | 1 | n.n | n.n | n.n |
| Salmonellen | 1 | n.n | n.n | n.n |

| | | | | |
|---|---|---|---|---|
| n.n: nicht nachgewiesen, NG: Nachweisgrenze | | | | |

Ergebnis:
Bei allen untersuchten Proben sowohl bei Raumtemperatur als auch bei 40 °C wurden keine Keime nachgewiesen. Über die sechs Monate hinaus wurden auch nach 12 Monaten mikrobiologische Test durchgeführt, aufgrund der veränderten Viskosität mussten die Proben 1:10 verdünnt werden, somit lag die Nachweisgrenze bei 10 und es konnten auch nach dieser Zeit keine Keime im Solubilisat nachgewiesen werden.

### Beispiel 3:

### Stabilitätstest - Alterungstest am Beispiel von Propolis-Solubilisat

Für den Alterungstest wurden Proben von Propolis-Solubilisat bei Raumtemperatur und 40 °C gelagert. Es wurden Proben an folgenden Zeitpunkten gezogen: Tag 0, 30, 90 und 270. Dabei wurden folgende Parameter bestimmt. Farbe, Trübung, pH-Wert, Dichte und Sediment. Da Propolis von Natur aus eine dunklere Farbe aufweist, wurden die Proben 1: 10 mit Wasser verdünnt, um Trübungen zu erkennen und den pH Wert zu bestimmen.

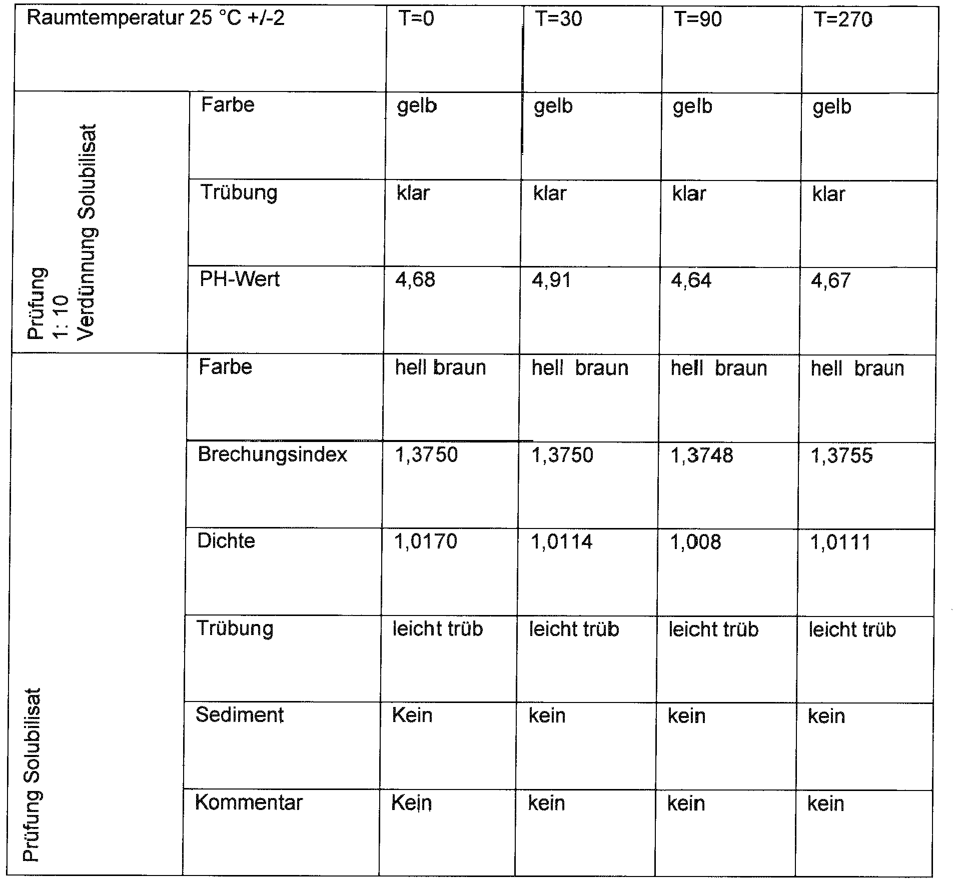

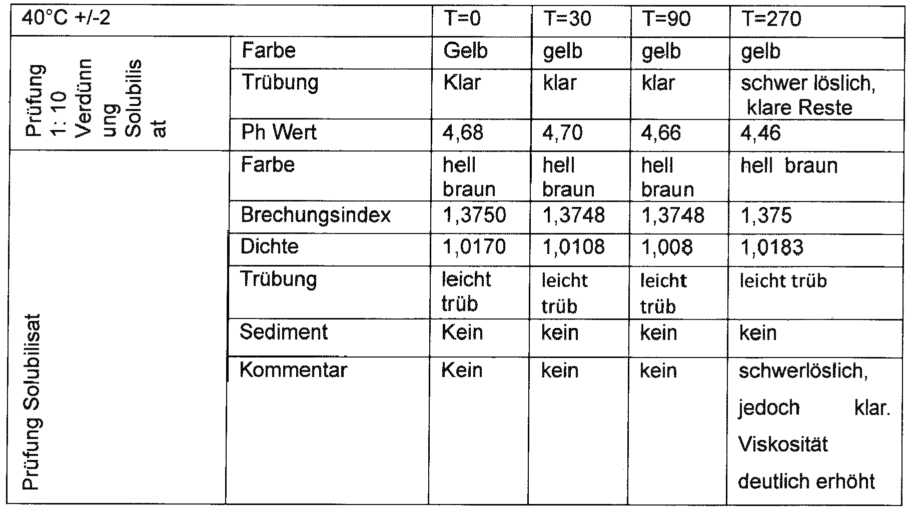

### Beispiel 4:

### Stabilitätstest UV-Licht bei Raumtemperatur

Methode:
Cannabinoide sind besonders empfindlich gegenüber Licht, das die Bildung spezifischer Oxidationsprodukte hervorruft. Daher wurde ein HPLC-Experiment durchgeführt, um den Unterschied in der Stabilität von CBD in wässrig-organischer Lösung (Acetonitril/Wasser) im Vergleich zu einer 1 Gew.-%igen CBD-Formulierung mit Poloxamer zu untersuchen.

Die Proben wurden bis zu 281 Tage dem natürlichen Tageslicht-Zyklus ausgesetzt und in regelmäßigen Abständen gemessen. Als Negativkontrolle wurden gleiche formulierte Proben im Dunkeln und bei 4 °C gelagert.

### Herstellung der CBD Acetonitril/Wasserlösung SOL031-TS-010 Stammlösung:

Es wurden 109,9 mg Cannabidiol (Reinheit 97 %) im 100 ml Messkolben eingewogen und mit Acetonitril /H₂O (60/40) aufgefüllt.

### Aufarbeitung der Proben:

100 µl Probe +900 µl Acetonitril (ACN) werden in ein Reaktionsröhrchen überführt und mittels Vortex gut vermischt ("gevortext"), anschließend in ein HPLC Fläschchen umgefüllt und an der HPLC gemessen.

### Herstellung der Poloxamer-Formulierung SOL031-46

Es wird 1 % Cannabidiol-Solubilisat mit Poloxamer hergestellt, entspricht 10 mg /ml Cannabidiol

### Aufarbeitung der Probe:

100 µl Probe + 1900 µl MeOH/Acetonitril (1:1) wurden in einem 50 ml Röhrchen 1 h extrahiert. Anschließend wird die Probe zentrifugiert, 100 µl Überstand abgenommen und mit 900 µl Acetonitril vermengt und an der HPLC gemessen.

### HPLC Messmethode:

Laufmittel: 90 % ACN + 10 % Wasser+ 0,1 % Ameisensäure, Isokratisch Säulenofen Temperatur: 38°C
Sampler Temperatur: 38 °C
Flow: 0,5 ml/min
Laufzeit: 15 min
Wellenlänge: 280 nm

Es wurden HPLC-Chromatogramme der CBD-Lösung mit Acetonitril und Wasser (1 mg/ml) und der CBD-Poloxamer Formulierung (1 w/w-% ) aufgenommen.

### Ergebnis

Die Poloxamer-Formulierung zeigt eine höhere Stabilität von CBD im Vergleich zur wässrig-organischen (ACN/H₂O) Lösung. Nach mehr als 275 Tagen Exposition gegenüber dem natürlichen Tageslichtzyklus bei Raumtemperatur ist die CBD-Konzentration in der wässrig-organischen Lösung auf 3,7 % der Ausgangsmenge gesunken (die Dunkelheitskontrolle auf 80 %). Im Vergleich dazu ist die CBD-Konzentration in der Formulierung mit Poloxamer auf 50,4 % reduziert (die Dunkelkontrolle zeigte keinerlei Reduzierung von CBD).

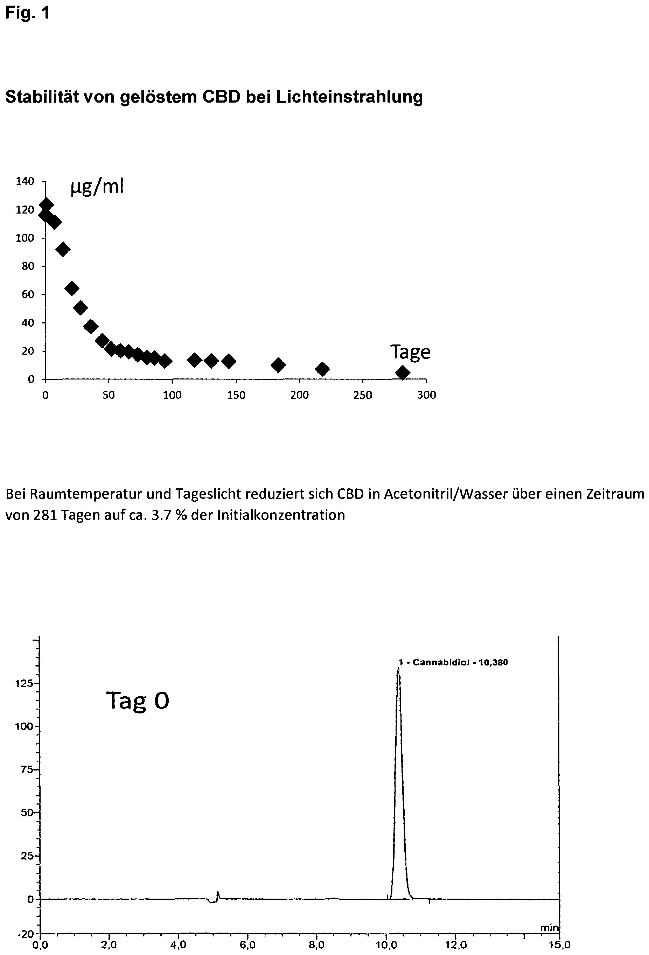

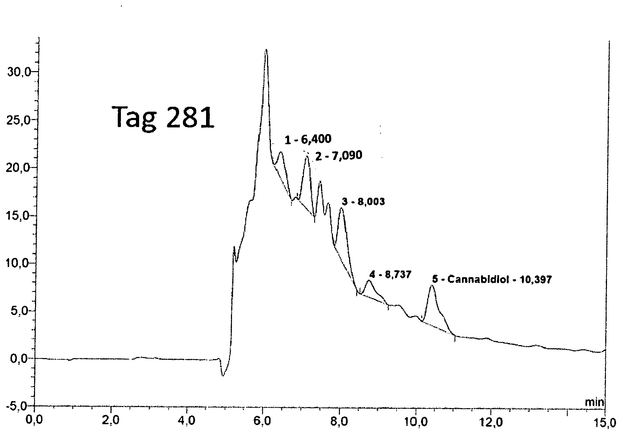

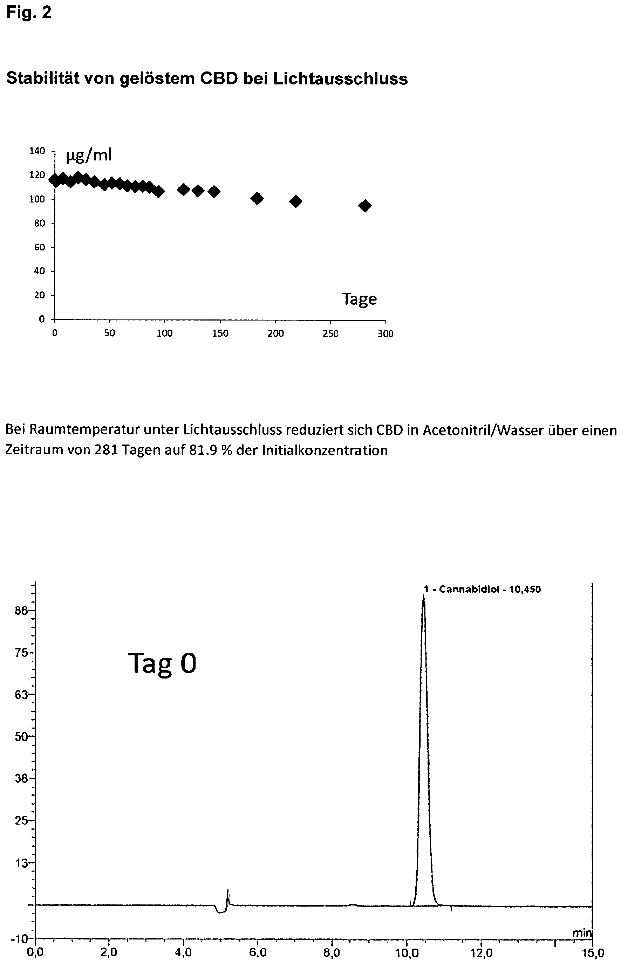

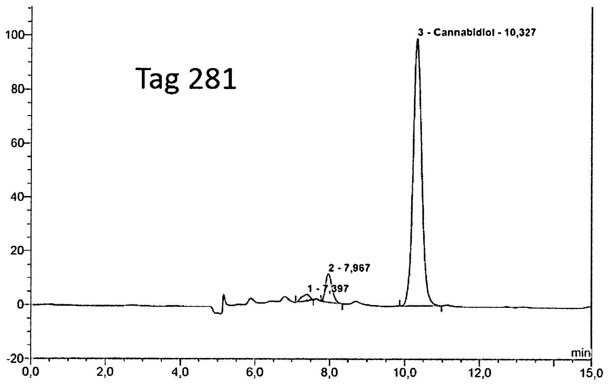

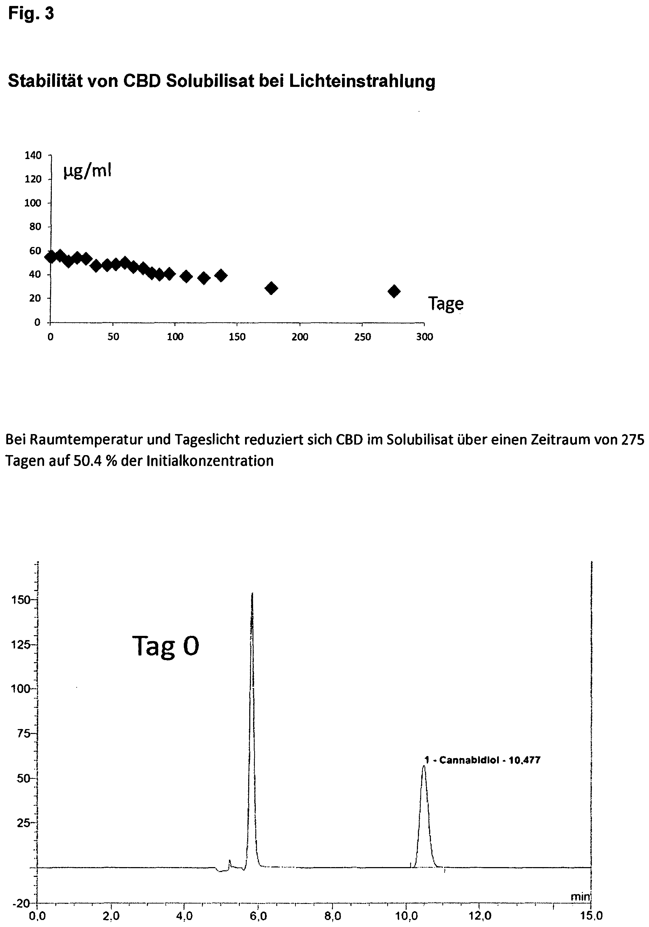

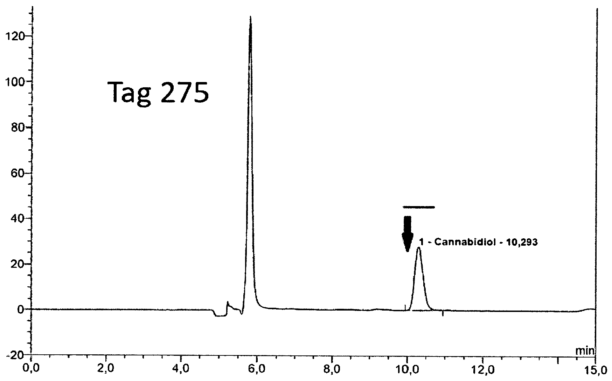

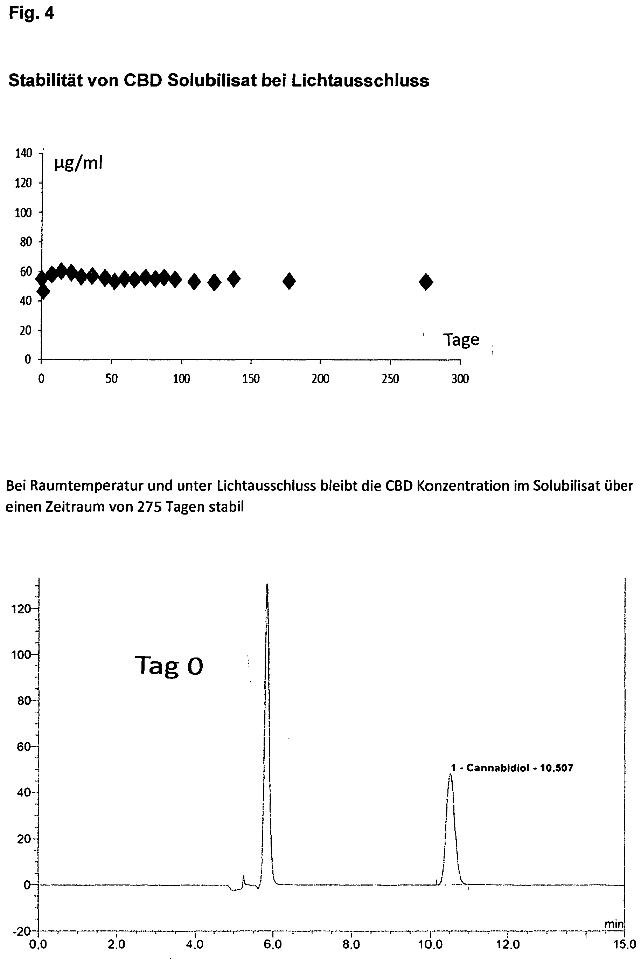

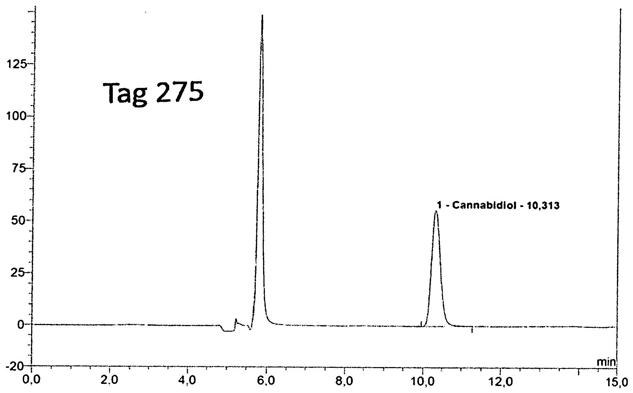

Die Ergebnisse sind in den Fig. 1 - 4 gezeigt.

### Beispiel 5:

### Stabilität CBD-Solubilisat und CBD-Isolat im Kosmetikprodukt

### Tagescreme mit CBD-Isolat oder CBD-Solubilisat

Um die Stabilität des Solubilisates im kosmetischen Produkt zu vergleichen, wurden verschiedene Tagescreme- Formulierungen (s. Tabelle) mit CBD-Solubilisat und CBD-Isolat hergestellt. Die Hauptkomponente der Creme blieben dabei gleich, es wurde die Konzentration der zusätzlichen Stabilisatoren verändert und deren Auswirkung auf dem CBD Gehalt beobachtet.

Bestandteile (Ingredienzen):
Wasser, Propylenglycol, Jojobaöl, Aprikosenkernöl, Harnstoff (Urea), Natriumlactat, Sheabutter, Kokos, beta-Glucan, Pektin, Zedernussöl, Methylglucosesesquistearat, Lanolin, Aloe Vera-Pulver, Milchsäure, Cetylalkohol, Myristylmyristat, Hyaluronsäure, Xanthan, Kaliumsorbat, CBD-Isolat oder CBD Solubilisat und /oder Vitamin E, Vitamin C

Die Tagescreme besteht aus über 50% wässrigen Anteil, 21% Fettphase, die den Emulgator bereits miteinschließt, 0,1 % Cannabidiol-Isolat oder 1 % Cannabidiol-Solubilisat sowie weiteren Wirkstoffen.

Die Herstellung der Tagescreme:
Nach Aufschmelzung der Fettphase wird diese mit der Wasserphase emulgiert, die weiteren Wirkstoffe werden eingearbeitet.

Der Unterschied in der Herstellung besteht in der Zugabe der jeweiligen Cannabidiole. Während das CBD-Isolat in der Fettphase aufgeschmolzen wird, wird das CBD-Solubilisat in der Wasserphase gelöst.

### Berechnete CBD Konzentration im Endprodukt

Bei Zugabe von 0,1% CBD Isolat ergibt es eine Endkonzentration im Produkt von 0,1 % Cannabidiol.

Bei Zugabe von 1% CBD-Solubilisat (1% Cannabidiol im Solubilisat) ergibt es eine Endkonzentration im Produkt von 0,01 % Cannabidiol.

Die hergestellten Proben wurden im Klimaschrank bei Raumtemperatur und 40 °C gelagert.

### Aufarbeitung der Proben

### Tagescreme mit 0,1 % CBD Isolat

200 mg Creme werden in 10 ml Kolben eingewogen und THF (Tetrahydrofuran) aufgefüllt und mittels Vortex gemischt ("gevortext"). Nach mind. 12 Stunden Extraktion werden 200 µl Proben und 800 µl Acetonitril (ACN)/H₂O (70/30) im Reaktionsröhrchen gemischt und abzentrifugiert. Der Überstand wird in ein HPLC-Gefäß überführt und gemessen

### Tagescreme mit 1% Solubilisat

1000-2000 mg Creme werden in 10 ml Kolben eingewogen und THF (Tetrahydrofuran) aufgefüllt und "gevortext". Nach mind. 12 Stunden Extraktion werden 200 µl Proben und 800 µl ACN/H2O (70/30) im Reaktionstube gemischt und abzentrifugiert. Der Überstand wird in ein HPLC- Gefäß überführt und gemessen

Tagescreme 21 % Fettanteil (O/W) mit verschiedenen Stabilisatoren Konzentrationen Tagescreme mit CBD Isolat, 0,1% CBD:

| | | | |
|---|---|---|---|
| **CBD Tagescreme** | **CBD Isolat Ohne Vit E und Vit C** | **RT/40°C** | **COS013-AL-008** |
| **CBD Tagescreme** | **CBD Isolat 0,2 %Vit E und 0,2 % Vit C** | **RT/40°C** | **COS013-AL-009** |
| **CBD Tagescreme** | **CBD Isolat 1 %Vit E und 0,2 VIT C** | **RT/40°C** | **COS013-AL-010** |
| **CBD Tagescreme** | **CBD Isolat 1 %Vit E und 1% Vit C** | **RT/40°C** | **COS013-AL-011** |

**Tagescreme: mit CBD Solubilisat, 0,01% CBD:**

| | | | |
|---|---|---|---|
| **CBD Tagescreme** | **CBD Solubilisat Ohne Vit E und Vit C** | **RT/40°C** | **COS013-AL-012** |
| **CBD Tagescreme** | **CBD Solubilisat 0,2% Vit Eund 0,2 %Vit C** | **RT/40°C** | **COS013-AL-013** |
| **CBD Tagescreme** | **CBD Solubilisat 1 %Vit E und 0,2 VIT C** | **RT/40°C** | **COS013-AL-014** |
| **CBD Tagescreme** | **CBD Solubilisat 1 %Vit E und 1% Vit C** | **RT/40°C** | **COS013-AL-015** |

### Ergebnis:

**CBD- Tagescreme Isolat**

| **Tagescreme** | **CBD Isolat Konzentration** % | **Temperatur** | **200 µl/10 ml Verdünnung (1/50) µg/ml** | **200µl/1000µl Verdünnung (1/5) µg/ml** | **t0 Abweichung (%)** | **Initial µg/ml** | **t90 Abweichung (%)** |
|---|---|---|---|---|---|---|---|
| **Proben No (F&E)** | | | | | | | |
| **Datum** | | | | | **27.09.2021** | **27.09.2021** | **22.12.2021** |
| COS013-AL-008 | 0,1 | RT | 20 | 4 | 128,65 | 5,15 | 93,27 |
| COS013-AL-008 | 0,1 | 40 | 20 | 4 | 128,65 | 5,15 | 90,49 |
| COS013-AL-009 | 0,1 | RT | 20 | 4 | 141,99 | 5,68 | 97,75 |
| COS013-AL-009 | 0,1 | 40 | 20 | 4 | 141,99 | 5,68 | 94,83 |
| COS013-AL-010 | 0,1 | RT | 20 | 4 | 115,92 | 4,64 | 96,01 |
| COS013-AL-010 | 0,1 | 40 | 20 | 4 | 115,92 | 4,64 | 91,81 |
| COS013-AL-011 | 0,1 | RT | 20 | 4 | 123,42 | 4,94 | 95,40 |
| COS013-AL-011 | 0,1 | 40 | 20 | 4 | 123,42 | 4,94 | 93,12 |

**CBD- Tagescreme-Solubilisat**

| **Tagescreme** | **CBD Solubilisat Konzentration 0,01 %** | **Temperatur** | **1000 µl/10 ml Verdünnung (1/10) µg/ml** | **200µl/1000µl Verdünnung (1/5) µg/ml** | **t0 Abweichung (%)** | **Initial µg/ml** | **t90 Abweichung (%)** |
|---|---|---|---|---|---|---|---|
| **Proben No (F&E)** | | | | | | | |
| **Datum** | | | | | **27.09.2021** | **27.09.2021** | **22.12.2021** |
| COS013-AL-012 | 1 | RT | 10 | 2 | 111,41 | 2,23 | 96,80 |
| COS013-AL-012 | 1 | 40 | 10 | 2 | 111,41 | 2,23 | 95,87 |
| COS013-AL-013 | 1 | RT | 10 | 2 | 110,47 | 2,21 | 97,45 |
| COS013-AL-013 | 1 | 40 | 10 | 2 | 110,47 | 2,21 | 95,59 |
| COS013-AL-014 | 1 | RT | 10 | 2 | 109,39 | 2,19 | 99,21 |
| COS013-AL-014 | 1 | 40 | 10 | 2 | 109,39 | 2,19 | 99,30 |
| COS013-AL-015 | 1 | RT | 10 | 2 | 108,13 | 2,16 | 95,23 |
| COS013-AL-015 | 1 | 40 | 10 | 2 | 108,13 | 2,16 | 98,08 |

Erläuterung zur Tabelle:
"t0"/"Initial"/"t90": Während des Herstellprozesses können minimale Einwaagefehler, Abdampfung, Verluste durch Rührwerke zu Abweichungen an CBD-Konzentration in Endprodukt führen. Es gilt, je kleiner die eine Charge desto größer die Abweichung. Deshalb muss die tatsächliche Konzentration im Endprodukt am Tag 0 bestimmt werden. Dieser Initialwert dient als Startwert. Am Tag 0 wird die Abweichung von Sollwert bestimmt. An Tag 90, wird die Abweichung von Initialwert berechnet.

**Gegenüberstellung von Tagescreme mit CBD-Isolat und CBD-Solubilisat (es werden nur die 40 °C Proben gegenübergestellt)**

| **Stabilisatoren** | **Solubilisat 0,01% CBD** | **COS Nr. 40°C** | **CBD Isolat 0,1 % CBD** | **COS Nr. 40°C** |
|---|---|---|---|---|
| **Ohne Vit E und Vit C** | 95,87 % | COS013-AL-012 | 90,49 % | COS013-AL-008 |
| **0,2% Vit E und 0,2 % Vit C** | 95,59 % | COS013-AL-013 | 94,83 % | COS013-AL-009 |
| **1% Vit E und 0,2 % Vit C** | 99,3% | COS013-AL-014 | 91,81 % | COS013-AL-010 |
| **1% Vit E und 1% Vit C** | 98,08 % | COS013-AL-015 | 93,12 % | COS013-AL-011 |

Die Messung zeigt, dass ohne Zugabe von Stabilisatoren (Vitamin E und Vitamin C) das CBD in der Mizelle (COS013-AL-012) eine etwas höhere Stabilität nach 90 Tagen aufweist als die gleichartige Tagescreme mit CBD-Isolat (COS013-AL-008).

Beim Einsatz von Stabilisatoren, d.h. Vitamin E und C in der gleichwertigen niedrigen Menge (z.B. 0,2%), ist bei beiden Formulierungen (COS013-AL-013/COS013-AL-009) eine gleiche Stabilität der beiden Wirkstoffe zu beobachten.

Bei Zugabe von Vitamin E in höherer Konzentration (1%) ist eine deutliche Stabilität des Solubilisats zu beobachten als die Stabilität des Isolates (COS013-AL-014/COS013-AL-010). Dieser Effekt ist auch weiterhin zu beobachten in der zweiten Messung mit 1% Vitamin E plus 1% Vitamin C (COS013-AL-015/COS013-AL-011).

### CBD-Serum, 5 % Fettanteil

### Serum mit CBD-Isolat oder CBD-Solubilisat

Um die Stabilität des Solubilisats im kosmetischen Produkt zu vergleichen, wurden verschiedene Seren-Formulierungen (s. Tabelle) mit CBD-Solubilisat und CBD-Isolat hergestellt. Die Hauptkomponente der Serums blieben dabei gleich, es wurde die Konzentration der zusätzlichen Stabilisatoren verändert und deren Auswirkung auf dem CBD Gehalt beobachtet.

Bestandteile (Ingredienzen):
Rosenhydrolat, Wasser, Propylen, Jojobaöl, Lecithin, Betain, Hydroxyethylcellulose, Myristylmyristate, Kaliumsorbat, Xanthan, CBD-Isolat oder CBD- Solubilisat 1% und/oder Vitamin C, Vitamin E

Das Serum besteht aus 70% wässrigem Anteil, 5% Fettphase, Emulgator, 0,1 % Cannabidiol-Isolat oder 1 % Cannabidiol-Solubilisat sowie weiteren Wirkstoffen.

### Herstellung des CBD Serums

Nach Aufschmelzung der Fettphase wird diese mit der Wasserphase emulgiert, die weiteren Wirkstoffe werden eingearbeitet.

Der Unterschied in der Herstellung besteht in der Zugabe der jeweiligen Cannabidiole. Während das CBD-Isolat in der Fettphase aufgeschmolzen wird, wird das CBD-Solubilisat in der Wasserphase gelöst.

### Berechnete CBD-Konzentration im Endprodukt

Bei Zugabe von 0,1% CBD Isolat ergibt es eine Endkonzentration im Produkt von 0,1 % Cannabidiol.

Bei Zugabe von 1% CBD-Solubilisat (1% Cannabidiol im Solubilisat) ergibt es eine Endkonzentration im Produkt von 0,01 % Cannabidiol.

Die hergestellten Proben wurden im Klimaschrank bei Raumtemperatur und 40 °C gelagert.

### Aufarbeitung der Proben

### Serum mit 0,1 % CBD Isolat

800 mg Serum werden in 10 ml Kolben eingewogen und THF (Tetrahydrofuran) aufgefüllt und mittels Vortex gemischt ("gevortext"). Nach mind. 12 Stunden Extraktion werden 200 µl Proben und 800 µl Acetonitril(ACN)/H₂O (70/30) im Reaktionsröhrchen gemischt und abzentrifugiert. Der Überstand wird in ein HPLC-Gefäß überführt und gemessen

### Serum mit 1% Solubilisat

200-400 mg Serum werden in 2 ml Kolben eingewogen und THF (Tetrahydrofuran) aufgefüllt und "gevortext". Nach mind. 12 Stunden Extraktion werden 200 µl Proben und 800 µl ACN/H₂O (70/30) im Reaktionsröhrchen gemischt und abzentrifugiert. Der Überstand wird in ein HPLC-Gefäß überführt und gemessen

**COS011-AL CBD Serum mit 5% Fett**

| | **Konzentration CBD** | **Temperatur** | **200 µl/2 ml Verdünnung (1/10) µg/ml** | **200µl/1000µl Verdünnung (1/5) µg/ml** | **t0 Abweichung (%)** | **Initial µg/ml t= 30 Tage** | **t30 Abweichung (%)** | **t90 Abweichung (%)** |
|---|---|---|---|---|---|---|---|---|
| **Proben No (F&E)** | | | | | | | | |
| **Datum** | | | | | | | | |
| COS 011-AL-006. RT | 0,01 | RT | 9,81 | 1,96 | 90,10 | 2,02 | 100 | 106,22 |
| COS 011-AL-006.40°C | 0,01 | 40°C | 9,81 | 1,96 | 90,10 | 1,91 | 100 | 97,49 |
| Bemerkung: Initialwert ab Tag 30 | | | | | | | | |

**COS011-AL CBD Serum mit 5 % Fett**

| | **Konzentration CBD** | **Temperatur** | **400 µl/2 ml Verdünnung** | **200µl/1000µl Verdünnung** | **t0 Abweichung** | **Initial** | **t90 Abweichung** |
|---|---|---|---|---|---|---|---|
| **Proben No (F&E)** | | | **(1/5) µg/ml** | **(1/5) µg/ml** | **(%)** | **µg/ml** | **(%)** |
| **Datum** | | | | | **12.11.2021** | **12.11.2021** | **15.02.2022** |
| COS011-AL-030 | 0,01 | RT | 20 | 4 | 124,32 | 4,97 | 107,13 |
| COS011-AL-030 | 0,01 | 40°C | 20 | 4 | 124,32 | 4,97 | 64,14 |
| COS011-AL-031 | 0,01 | RT | 20 | 4 | 110,97 | 4,24 | 105,46 |
| COS011-AL-031 | 0,01 | 40°C | 20 | 4 | 110,97 | 4,24 | 91,32 |
| COS011-AL-032 | 0,01 | RT | 20 | 4 | 115,23 | 4,44 | 97,40 |
| COS011-AL-032 | 0,01 | 40°C | 20 | 4 | 115,23 | 4,24 | 28,46 |
| | | | | | **t=0** | **Initial** | **t90** |

| | **Konzentration CBD** | **Temperatur** | **800 µl/10 ml Verdünnung** | **200µl/1000µl Verdünnung** | **Abweichung** | | **Abweichung** |
|---|---|---|---|---|---|---|---|
| **Proben No (F&E)** | | | **(1/12,5) µg/ml** | **(1/5) µg/ml** | **(%)** | **µg/ml** | **(%)** |
| **Datum** | | | | | **12.11.2021** | **12.11.2021** | **15.02.2022** |
| COS011-AL-033 | 0,1 | RT | 80 | 16 | 116,51 | 18,64 | 101,95 |
| COS011-AL-033 | 0,1 | 40°C | 80 | 16 | 116,51 | 18,64 | 47,68 |
| COS011-AL-034 | 0,1 | RT | 80 | 16 | 122,00 | 19,52 | 99,27 |
| COS011-AL-034 | 0,1 | 40°C | 80 | 16 | 122,00 | 19,52 | 92,96 |
| COS011-AL-035 | 0,1 | RT | 80 | 16 | 105,91 | 16,95 | 93,37 |
| COS011-AL-035 | 0,1 | 40°C | 80 | 16 | 105,91 | 16,95 | 63,19 |

| **Stabilisatoren** | **Solubilisat 0,01% CBD** | **COS Nr. 40°C** | **Emulsion** | **CBD Isolat 0,1 % CBD** | **COS Nr. 40°C** | **Emulsion** |
|---|---|---|---|---|---|---|
| **Ohne Vit E und Vit C** | 97,49 % | COS011-AL-006 | Keine Trennung, weiß | **NA** | **NA** | **NA** |
| **0,2% Vit E und 0,2 % Vit C** | 64,14 % | COS011-AL-030 | Leichte Trennung, leicht gelb | 47,68 % | COS011-AL-033 | Mittlere Trennung, leicht gelb |
| 1**% Vit E und 0,2 % Vit C** | 91,32 % | COS011-AL-031 | Leichte Trennung, leicht gelb | 92,96 % | COS011-AL-034 | Leichte Trennung, leicht gelb |
| **1% Vit E und 1% Vit C** | 28,46 % | COS011-AL-032 | Leichte Trennung, gelb | 63,19 % | COS011-AL-035 | Starke Trennung, gelb |

Es wurde festgestellt, dass bei Emulsionen mit CBD-Isolat, mit 5 % Fettanteil, durch die Zugabe von Vitamin C die Stabilität der Formulierung beeinträchtigt wird. Je höher der Vitamin C-Anteil, desto stärker ist die Trennung der Emulsion. Dieser Trennungsprozess ließ sich nur mit hoher Konzentration an Vitamin E (1%) im Serum verringern.

Dagegen wurde keine mittlere oder starke Trennung der Emulsion bei den Formulierungen mit CBD-Solubilisat im Serum in dem Zeitraum von drei Monaten bei 40 Grad Celsius beobachtet. Daher lässt sich schließen, dass die Formulierungen mit Solubilisaten stabiler sind, unabhängig von Stabilisatoren und deren Konzentration, als die mit CBD-Isolat.

## Patentansprüche

1. Topische Zusammensetzung aufweisend einen oder mehrere Solubilisate von lipophilen Wirkstoffen aus Pflanzenauszügen, Pflanzenextrakten, Pflanzenteilen, Phytotherapeutika, Harzen, Enzymen, Vitaminen, Proteinen, Algen, funktionellen Aktivstoffen und/oder Coenzymen sowie Mischungen und Kombinationen davon.

2. Zusammensetzung nach Anspruch 1, wobei die Pfanzenauszüge, Pflanzenextrakte bzw. Phytotherapeutika aus Nachtkerze (Oenothera biennis), Ballonrebe (Cardiospermum halicacabum), Wildes Stiefmütterchen (Viola tricolor), Zinnkraut (Equisetum arvense), Echte Kamille (Matricaria recutita), Zauberstrauch (Hamamelis virginiana), Mahonie (Mahonia aquifolium), Birke (Betula pendula), Ylang-Ylang, Sibirischer Ginseng (Eleutherocokk), Sheabutter (Karitebutter), Cannabispflanze (Cannabinoide), Kurkuma (Curcuma Ianga), Echter Lavendel (Lavandula angustifolia), Johanniskraut (Hypericum perforatum), Irisches Moos (Chondrus Crispus), Hopfen (Humulus Lupulus) Grüntee (Camellia sinensis L.), Centella ( Centella asiatica), Malve (Malva sylvestris), Mädesüß (Filipendula ulmaria), Ringelblume (Calendula officinalis L.), Rotklee (Trifolium pratense) stammen.

3. Zusammensetzung nach Anspruch 1 oder 2, wobei der Wirkstoff Cannabidiol (CBD) ist.

4. Zusammensetzung nach Anspruch 1, wobei die Enzyme, Proteine bzw. Coenzyme Lipasen, Cellulasen, Amylasen, Proteasen, Kollagen, Acetyl Hexapeptid-8, Pentapeptid-18 und/oder Ubichinon-10 (Q10) sind.

5. Zusammensetzung nach Anspruch 1, wobei die Harze Weihrauch, Propolis oder Myrrhe sind.

6. Zusammensetzung nach Anspruch 1, wobei die funktionellen Aktivstoffe Astaxanthin, Resveratrol, Pterostilbene, Fruchtsäuren oder Substanzen sind, die mit den Funktionswegen von Hypoxie-induziertem Faktor (HIF), mTor, p53, GDNF, GLUT1 interagieren können.

7. Zusammensetzung nach einem der Ansprüche 1-6, wobei die Solubilisate eine Kombination von Emulgator(en), fettlöslichen Wirkstoffen und Hilfsstoffen darstellen.

8. Zusammensetzung nach Anspruch 7, wobei der Emulgator aus Polysorbaten, Lutrol und/oder D-alpha-Tocopheryl Polyethylenglycol 1000 Succinat ausgewählt ist.

9. Zusammensetzung nach Anspruch 7 oder 8, wobei die Hilfsstoffe Ethanol, Polysorbat und/oder Glycerin sind.

10. Zusammensetzung nach einem der Ansprüche 1-9, wobei sie zusätzlich kosmetische bzw. pharmazeutische Hilfsstoffe, vorzugsweise Emulgatoren, Stabilisatoren, Konservierungsmittel, Verdickungsmittel und/oder Bindemittel enthält.

11. Zusammensetzung nach Anspruch 10, wobei der Stabilisator Vitamin C oder Vitamin D ist.

12. Zusammensetzung nach einem der Ansprüche 1-11, wobei es sich um eine Creme, Lotion, Milch, Serum, Gel, Tropfen, Emulsion, Balsam, Fluid, Salbe, Maske und/oder Schaum handelt.

13. Verwendung der Zusammensetzung nach einem der Ansprüche 1-10 für kosmetische Zwecke zur Pflege und/oder Reinigung jeglichen Hauttyps.

14. Zusammensetzung nach einem der Ansprüche 1-12 zur Verwendung in einem Verfahren zur Behandlung von Hauterkrankungen.

15. Zusammensetzung zur Verwendung nach Anspruch 14, wobei die Hauterkrankung Akne, Herpes, Neurodermitis, Urtikaria, Erysipel, Verbrennungen oder Psoriasis ist.
